Europäisches Patentamt

(19) European Patent Office

Office européen des brevets

(11) Publication number: **0 303 308 B1**

(12) **EUROPEAN PATENT SPECIFICATION**

(45) Date of publication of patent specification :
29.05.91 Bulletin 91/22

(51) Int. Cl.⁵: **A61K 37/26, A61K 9/22**

(21) Application number: 88201499.6

(22) Date of filing: 13.07.88

(54) Preparation for preventing or combating complications in diabetes.

(30) Priority: 14.08.87 NL 8701912

(43) Date of publication of application:
15.02.89 Bulletin 89/07

(45) Publication of the grant of the patent:
29.05.91 Bulletin 91/22

(84) Designated Contracting States:
AT BE CH DE FR GB IT LI NL SE

(56) References cited:
EP-A- 0 179 332
CH-A- 330 318
GB-A- 2 104 382
US-A- 2 789 080
US-A- 3 842 064
US-A- 4 492 684

(73) Proprietor: AKZO N.V.
Velperweg 76
NL-6824 BM Arnhem (NL)

(72) Inventor: Huisman, Robby
Gasthuisstraat 11
NL-5361 HH Grave (NL)

(74) Representative: Hermans, Franciscus G.M. et al
Patent Department AKZO N.V. Pharma Division P.O. Box 20
NL-5340 BH Oss (NL)

Jouve, 18, rue Saint-Denis, 75001 PARIS

## Description

The present invention relates to a pharmaceutical preparation for combating or preventing complications in diabetes mellitus.

Diabetes mellitus is primarily characterized by a reduction or cessation of the ability to oxidize carbohydrates, resulting in partial or complete disturbance of the normal insulin mechanism.

Furthermore, other complications regularly arise in diabetes mellitus patients, especially after a longer period, which can be of a sensory, autonomous or, to a lesser extent, motor type. These complications, in general also called diabetic neuropathies, relate to a very heterogeneous and frequently also mutually overlapping group of neurological syndromes, and as rule arise not withstanding the fact that the blood sugar level is kept within reasonable limits by treatment with insulin. In a number of cases the abovementioned complications have proved irreversible, so that the patient naturally benefits if the complications in question do not arise at all.

Sensory complications often manifest themselves in the form of pain, autonomous complications in the form of, inter alia, organic impotence and orthostatic hypotension, and motor complications in the form of muscular weakness.

Some of these complications can be eliminated with reasonable success by a highly balanced and excellently controlled administration of insulin. However, such treatment is in many cases not possible and is in any case highly patient-unfriendly.

According to some experts, a possible cause of these neurological complications in diabetes mellitus is thought to be the formation of sorbitol, resulting from glucose with the aid of the enzyme aldose reductase. Hence, the administration of aldose reductase inhibitors is proposed as a possible method of treatment for combating these diabetes mellitus complications

The present invention provides a pharmaceutical preparation for combating or preventing complications in patients with diabetes, which keeps the blood sugar level within reasonable limits and at the same time combats or prevents the said neurological complications.

The preparation according to the invention comprises :

(a) insulin or a salt or complex thereof, and

(b) a peptide of the general formula I : H-L-Met(X)-L-Glu-L-His-L-Phe-D-Lys-L-Phe-Y (I)

or a salt or a N-acyl derivative thereof, wherein Met(X) represents the amino acid radical Met, Met(O) or Met($O_2$),

Y represents the group Gly-Z or Z, and

Z represents the hydroxyl group, an esterified hydroxyl group or a substituted or unsubstituted amino group.

As the insulin, it is in principle possible to use any insulin, crystalline or amorphous, insulin of animal origin or produced with the aid or recombinant DNA techniques or by synthetic or semisynthetic methods of preparation.

Depending on the requirements of the individual patient it is, moreover, possible to choose a quick-acting insulin form or slower-acting insulin forms, with the latter in general consisting essentially of insulin-zinc complexes or protamine-insulin complexes.

Peptides of the general formula I are known peptides. Concerning the preparation of such peptides reference is made, for brevity, to U.S. Patent 3,842,064 and European Patent Application 179,332. These peptides are in particular known for their psychopharmacological action.

Peptides according to formula I which are preferably used within the scope of the present invention, are those wherein Met(X) represents the amino acid Met($O_2$), (methioninesulphone) and Y represents a hydroxyl group, a hydroxyl group which is esterified with an aliphatic alcohol having 1 to 18 carbon atoms (and preferably a lower aliphatic alcohol having 1 to 6 carbon atoms) or a substituted amino group of the type –NH-ALK-NH$_2$, wherein ALK represents an alkylene group with 2 to 10 carbon atoms, preferably 4 to 10 and more especially 7 or 8 carbon atoms.

A peptide having the formula II :

H-L-Met($O_2$)-L-Glu-L-His-L-Phe-D-Lys-L-Phe-OH (II)

or a salt thereof is more especially preferred within the scope of the present invention.

N-acyl derivatives of the peptides of formula I refers to peptides I which are acylated at the N-terminal side with a lower (1-8 C) aliphatic monocarboxylic acid or dicarboxylic acid, such as acetic acid, propionic acid, butyric acid, malonic acid, succinic acid or glutaric acid.

The pharmaceutical preparation according to the invention is preferably administered subcutaneously or intranasally.

For subcutaneous administration in the form of an intramuscular or intravenous injection preparation, the two peptides concerned, namely insulin and the peptide according to formula I, are dissolved, suspended or emulsified in a liquid suitable for injection. A solution of the two peptides in water is, however, preferred in this context.

For subcutaneous administration in the form of an infusion, the peptides are dissolved, at a concentration suitable for infusion, in a pharmaceutically acceptable solvent, preferably water. Such a solution of the peptides in question can be administered in so-called osmotic insulin pumps or insulin pumps working by different mechanisms.

An outstandingly suitable subcutaneous administration form is furthermore a so-called implant, a subcutaneous depot from which an effective quantity of the two peptides is released in a controlled manner and over a lengthy period. By implant there is here understood a subcutaneous administration form, wherein the peptides in question are dispersed in a suitable polymer or are encapsulated by such a polymer, and wherein the peptides are present in a quantity which is greater than is necessary for a normal injection dosage.

Very suitable implants which can function as subcutaneous depots are the so-called microcapsules, microspheres or microgranules, which can be conveniently injected subcutaneously with the aid of a liquid. Larger shapes such as granules or "rods" can also be used. The larger dimensions and thus more troublesome introduction procedure of the latter depot forms are often more than compensated for by the convenient removablity of this type of implant.

Suitable polymers which can be used for these implants are, inter alia, polysiloxanes, polyalkylenes such as polyethylene and polypropylene and above all the so-called biological degradable polymers among which the polylactides (including copolymers thereof) occupy a leading place. Methods for producing such implants are extensively described in the literature ; for brevity, reference is made in this context to the content of, for example, U.S. Patent 3,773,919 and European Patents 52,510 and 58,481.

It is furthermore obvious that a subcutaneous implant according to the present invention is not only relevant to a polymer wherein both peptides (insulin and the peptide according to formula I) are contained, but also – and even preferably – is relevant to a mixture of two types of microcapsules or other forms, wherein exclusively insulin is contained in one type and exclusively the peptide according to formula I is contained in the other type.

For intranasal administration, the two peptides in question are introduced into a medium suitable for intranasal administration and are administered by means of a propellant gas or spray mechanism.

The dosages of the two peptides in the preparation according to the invention depend greatly on the individual requirement of the diabetic patient.

In general, however, the subcutaneous injection preparation according to the invention contains between 10 and 100 international units of insulin per ml and preferably 20, 30 or 40 international units per ml.

The amount of the peptide according to the general formula I depends on the duration of action of the chosen insulin, but this peptide is preferably present in this preparation in a quantity of $10^{-4}$ to $2 \times 10^{-2}$ mmol per ml and more especially between $0.4 \times 10^{-3}$ and $7.5 \times 10^{-3}$ mmol per ml.

An outstandingly suitable injection preparation according to the invention contains between 20 and 45 international units of insulin per millilitre and between 0.35 and 6.5 mg of the peptide according to formula II (molecular weight 870) per millilitre.

Whenever the molecular weight of the peptide I to be administered is higher or lower than that of the abovementioned peptide II, the stated preferred dosage must then be altered correspondingly.

The amount of the injection preparation according to the invention to be administered daily depends largely on the individual condition of the patient, but will in general vary between 0.5 ml and 2 to 3 ml per day.

It is obvious that for infusion liquids and especially for implants different quantities of peptide have to be used. These quantities are, however, generally so chosen that the dosage of the two peptides in question which is to be administered daily broadly corresponds to the daily dosage described above (of the peptides in question) when administered in the form of one or more injections.

In addition to the abovementioned peptides, the preparation according to the invention can also contain other substances, in particular :

– antimicrobial substances, such as methyl parahydroxybenzoate,
– substances for rendering the preparation according to the invention isotonic, and
– substances which can adjust the pH of an aqueous solution of the preparation according to the invention.

Furthermore, the pH is preferably adjusted to a value of between 7 and 7.8 ; a slightly acidic pH can, however, also be readily used.

The preparation according to the invention is further illustrated by reference to the following example.

A solution containing the following per millilitre is prepared :

| The peptide of formula II | | 3.0 | mg |
| Insulin | | 40 | IU |
| Methyl parahydroxybenzoate | | 1 | mg |
| Sodium acetate trihydrate | | 1.36 | mg |
| Sodium chloride | | 7 | mg |
| Hydrochloric acid and sodium hydroxide | to give pH | 7.35 | |
| Water for injection | to give | 1 | ml |

## Claims

1. Preparation for preventing or combating complications in diabetes, characterized in that it comprises :
(a) insulin or a salt or complex thereof, and
(b) a peptide of the general formula I : H-L-Met(X)-L-Glu-L-His-L-Phe-D-Lys-L-Phe-Y
or a salt or a N-acyl derivative thereof, wherein Met(X) represents the amino acid radical Met, Met(O) or Met(O$_2$),
Y represents the group Gly-Z or Z, and
Z represents the hydroxyl group, an esterified hydroxyl group or a substituted or unsubstituted amino group.

2. Preparation according to claim 1, characterized in that the peptide according to formula I which is used is a peptide of formula II : H-L-Met(O$_2$)-L-Glu-L-His-L-Phe-D-Lys-L-Phe-OH
or a salt thereof.

3. Preparation according to claim 1 or 2 suitable for subcutaneous injection.

4. Preparation according to claim 1 or 2 suitable as an infusion preparation.

5. Preparation according to claim 1 or 2 suitable as a subcutaneous implant.

6. Implant according to claim 5 in the form of microcapsules, microspheres or microgranules.

7. Implant according to claim 6, characterized in that it consists of a mixture of microcapsules, microspheres or microgranules which contain insulin and microcapsules, microspheres or microgranules which contain the peptide according to formula I or a derivative thereof.

8. Subcutaneous injection preparation according to claim 3, characterized in that it contains 10-100 international units of insulin per ml and 10$^{-4}$ to $2 \times 10^{-2}$ mmol per ml of the peptide according to formula I or a salt or N-acyl derivative thereof.

9. Preparation according to claim 8, characterized in that it contains, per ml, 40 international units of insulin and 0.35 to 6.5 mg of the peptide of formula II (molecular weight 870).

## Ansprüche

1. Präparat zur Verhütung oder Bekämpfung von Komplikationen bei Diabetes, dadurch gekennzeichnet, dass es enthält :
(a) Insulin oder ein Salz oder einen Komplex davon und
(b) ein Peptid der allgemeinen Formel I :
H-L-Met(X)-L-Glu-L-His-L-Phe-D-Lys-L-Phe-Y
oder ein Salz oder ein N-Acylderivat davon, in welcher Met(X) den Aminosäurerest Met, Met(O) oder Met(O$_2$) bedeutet,
Y die Gruppe Gly-Z oder Z bedeutet und
Z die Hydroxylgruppe, eine veresterte Hydroxylgruppe oder eine substituierte oder unsubstituierte Aminogruppe bedeutet.

2. Präparat nach Anspruch 1, dadurch gekennzeichnet, dass das Peptid gemäss der Formel I, welches verwendet wird, ein Peptid der Formel II :
H-L-Met(O$_2$)-L-Glu-L-His-L-Phe-D-Lys-L-Phe-OH oder ein Salz Davon ist

3. Präparat nach Anspruch 1 oder 2, welches geeignet ist für die subcutane Injektion.

4. Präparat nach Anspruch 1 oder 2, welches geeignet ist als Infusionspräparat.

5. Präparat nach Anspruch 1 oder 2, welches geeignet ist als subcutanes Implantat.

6. Implantat gemäss Anspruch 5 in der Form von Microkapseln, Microkügelchen oder Microkörnern.

7. Implantat nach Anspruch 6, dadurch gekennzeichnet, dass es aus einem Gemisch von Microkapseln, Microkügelchen oder Microkörnern besteht, welche Insulin enthalten und Microkapseln, Microkügelchen oder Microkörner, welche das Peptid der Formel I oder ein Derivat davon enthalten.

8. Subcutanes Injektionspräparat nach Anspruch 3, dadurch gekennzeichnet, dass es 10-100 internationale Einheiten Insulin pro ml und $10^{-4}$ bis $2 \times 10^{-2}$ Millimol pro ml des Peptides der Formel I oder eines Salzes oder N-Acylderivates davon enthält.

9. Präparat nach Anspruch 8, dadurch gekennzeichnet, dass es pro ml 40 internationale Einheiten Insulin und 0,35 bis 6,5 mg des Peptides der Formel II (Molekulargewicht 870) enthält.

## Revendications

1. Préparation pour prévenir ou combattre les complications du diabète, caractérisée par le fait qu'elle contient :

(a) de l'insuline ou un sel ou complexe de celle-ci, et

(b) un peptide de formule générale I

H-L-Met(X)-L-Glu-L-Hi-L-Phe-D-Lys-L-Phe-Y (I) ou un sel ou un dérivé N-acylé de celui-ci, où Met(X) représente le radical d'acide aminé Met, Met(O) ou Met($O_2$),

Y représente le groupe Gly-Z ou Z, et

Z représente le groupe hydroxyle, un groupe hydroxyle estérifié ou un groupe amino substitué ou non substitué.

2. Préparation selon la revendication 1, caractérisée par le fait que le peptide selon la formule I qui est utilisé est un peptide de formule II :

H-L-Met($O_2$)-L-Glu-L-His-L-Phe-D-Lys-L-Phe-OH (II) ou un sel de celui-ci

3. Préparation selon la revendication 1 ou 2, convenant à l'injection sous-cutanée.

4. Préparation selon la revendication 1 ou 2, convenant comme préparation pour perfusion.

5. Préparation selon la revendication 1 ou 2, convenant comme implant sous-cutané.

6. Implant selon la revendication 5, sous la forme de microcapsules, de microsphères ou de microgranules.

7. Implant selon la revendication 6, caractérisé par le fait qu'il consiste en un mélange de microcapsules, microsphères ou microgranules qui contiennent de l'insuline et de microcapsules, microsphères ou microgranules qui contiennent le peptide selon la formule I ou un dérivé de celui-ci.

8. Préparation pour injection sous-cutanée selon la revendication 3, caractérisée par le fait qu'elle contient 10 à 100 unités internationales d'insuline par ml et $10^{-4}$ à $2 \times 10^{-2}$ mmol par ml du peptide selon la formule I ou d'un sel ou dérivé N-acylé de celui-ci.

9. Préparation selon la revendication 8, caractérisée par le fait qu'elle contient, par ml, 40 unités internationales d'insuline et 0,35 à 6,5 mg du peptide de formule II (poids moléculaire 870).